(19) Europäisches Patentamt
European Patent Office
Office européen des brevets

(11) EP 2 019 668 B1

(12) **EUROPEAN PATENT SPECIFICATION**

(45) Date of publication and mention of the grant of the patent:
**01.09.2010 Bulletin 2010/35**

(21) Application number: **08726666.4**

(22) Date of filing: **10.03.2008**

(51) Int Cl.:
***A61K 9/20*** *(2006.01)*

(86) International application number:
**PCT/US2008/003169**

(87) International publication number:
**WO 2008/109170 (12.09.2008 Gazette 2008/37)**

(54) **PHARMACEUTICAL COMPOSITION COMPRISING CANDESARTAN CILEXETIL**

PHARMAZEUTISCHE ZUSAMMENSETZUNG MIT CANDESARTAN-CILEXETIL

COMPOSITION PHARMACEUTIQUE COMPRENANT DU CANDÉSARTAN CILEXÉTIL

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **08.03.2007 US 906150 P**

(43) Date of publication of application:
**04.02.2009 Bulletin 2009/06**

(73) Proprietor: **Teva Pharmaceutical Industries Ltd.**
**49131 Petah Tiqva (IL)**

(72) Inventors:
- **LEIBOVICI, Minutza**
**42312 Netanya (IL)**
- **KANARI, Itamar**
**44235 Kefar Sava (IL)**
- **FOX, Michael**
**67291 Tel Aviv (IL)**

(74) Representative: **Eder, Michael**
**df-mp**
**Fünf Höfe**
**Theatinerstrasse 16**
**80333 München (DE)**

(56) References cited:
**EP-A- 0 459 136**
**EP-A- 0 546 358**
**US-A1- 2006 165 806**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 019 668 B1

**Description**

FIELD OF INVENTION

The present invention encompasses pharmaceutical compositions comprising candesartan cilexetil and processes for preparing the same.

BACKGROUND OF THE INVENTION

Candesartan ("CNS") is a potent, long-acting, selective $AT_1$ subtype angiotensin II receptor antagonist. Candesartan is a useful therapeutic agent for treating circulatory system diseases such as hypertensive diseases, heart diseases (e.g. hypercardia, heart failure, cardiac infarction, etc.), strokes, cerebral apoplexy, and nephritis, among others. Candesartan meets the requirement of high potency but it is poorly absorbed when administered orally. Therefore, the prodrug candesartan cilexetil was developed. During absorption from the gastrointestinal tract candesartan cilexetil is rapidly and completely hydrolyzed to candesartan. The chemical name for candesartan is: 2-ethoxy-1-[[2'-(1H-tetrazol-5-yl)biphenyl-4-yl]methyl]-1H-benzimidazole-7-carboxylic acid. The chemical name for candesartan cilexetil is (+)-1-[[(cyclohexyloxy)carbonyl]oxy]ethyl-2-ethoxy-1-[[2'-(1H-tetrazol-5-yl)[1,1'biphenyl]-4-yl]methyl]-1H-benzimidazole-7-carboxylate.

Candesartan cilexetil is a white to off-white powder and is practically insoluble in water and in methanol. Although candesartan cilexetil contains an asymmetric center in the ester portion of the molecule, it is sold as the racemic mixture.

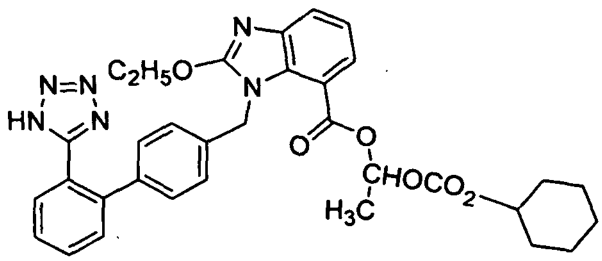

Candesartan Cilexetil

Angiotensin II is formed from angiotensin I in a reaction catalyzed by angiotensin-converting enzyme (ACE, kininase II). Angiotensin II is the principal pressor agent of the renin-angiotensin system, with effects that include vasoconstriction, stimulation of synthesis and release of aldosterone, cardiac stimulation, and renal reabsorption of sodium. Angiotensin II helps maintain constant blood pressure despite fluctuations in a person's state of hydration, sodium intake and other physiological variables. Angiotensin II also performs the regulatory tasks of inhibiting excretion of sodium by the kidneys, inhibiting norephedrine reuptake and stimulating aldosterone biosynthesis. Candesartan blocks the vasoconstrictor and aldosterone secreting effects of angiotensin II by selectively blocking the binding of angiotensin II to the $AT_1$ receptor in many tissues, such as vascular smooth muscle and the adrenal gland. By inhibiting angiotensin II binding to $AT_1$ receptors, candesartan disrupts the vasoconstriction mediated by $AT_1$ receptors. Blocking vasoconstriction by angiotensin II has been found to be beneficial to patients with hypertension.

The United States Food and Drug Administration has approved candesartan for the treatment of hypertension alone or in combination with other antihypertensive agents. Candesartan cilexetil is marketed in the United States under the trade name Atacand®. Atacand® tablets contain candesartan cilexetil and the following excipients: hydroxypropyl cellulose, polyethylene glycol, lactose, corn starch, carboxymethylcellulose calcium, and magnesium stearate.

Most tablet formulations also include common excipients such as binders, glidants (flow aids) and lubricants to ensure efficient tabletting. Typically, amino acids such as L-leucine have been used as a lubricant in tablets containing effervescent formulations of alendronate. For example, WO 97/044017 relates to pharmaceutical effervescent formulations of alendronate containing an acid source, a carbonate source, a binder, a lubricant and, optionally, flavoring agents, colorants and sweeteners, where one of the lubricants is L-leucine. Similarly, U.S. Patent No. 4,755,461 describes an improved tableted microconcentrated blood plasma coagulation reagent which comprises a combination of a first tablet containing thromboplastin, a buffer, a binder, and a lubricant; and a second tablet containing a calcium salt, where one of the lubricants is L-leucine.

European Publication No. EP0459136A describes a process for the preparation of candesartan cilexetil, and preparation of capsules/tablets comprising candesartan cilexetil.

European Publication No. EP0546358A relates to a method for preparing a pharmaceutical composition for oral use comprising admixing an effective amount of candesartan cilexetil with an oily substance having a lower melting point.

Candesartan cilexetil is observed to be stable against changes in temperature, moisture and/or light when it is stored alone in the solid state. However, the stability of candesartan cilexetil has been observed to suffer when candesartan cilexetil is incorporated into a pharmaceutical composition, especially a tablet, with other excipients. Not to be limited by theory, it is believed that the decrease in stability is brought about by deformation of crystals caused by, for example, pressure, abrasion and/or heat applied during the processes employed to prepare the compositions, e.g. granulation or molding under elevated temperature. Compression steps often employed in processes for preparing solid pharmaceutical compositions. For example, in the tablet-pressing process, it will often involve applying pressure, abrasion and/or heat to the composition constituents.

In view of the foregoing, there is a need to produce a stable pharmaceutical composition comprising candesartan cilexetil.

## SUMMARY OF THE INVENTION

In one embodiment, the present invention encompasses a pharmaceutical composition comprising candesartan cilexetil, at least one amino acid and a pharmaceutically acceptable excipient.

In another embodiment, the present invention encompasses a process of preparing a pharmaceutical composition comprising combining candesartan cilexetil, at least one amino acid and a pharmaceutically acceptable excipient.

In another embodiment, the present invention encompasses a process for stabilizing candesartan cilexetil in a pharmaceutical composition by adding at least one amino acid to the pharmaceutical composition having candesartan cilexetil.

In another embodiment, the present invention encompasses a method of treating a patient suffering from a disease comprising administering to a patient in need thereof a therapeutically effective amount of a pharmaceutical composition comprising candesartan cilexetil, at least one amino acid and a pharmaceutically acceptable excipient. Preferably, the disease is a circulatory system disease such as hypertension.

In another embodiment, the present invention encompasses use of candesartan cilexetil in the manufacture of a medicament for treating and/or preventing a patient suffering from a circulatory system disease comprising, at least one amino acid and a pharmaceutically acceptable excipient. Preferably, the medicament treats a circulatory system disease such as hypertension.

## DETAILED DESCRIPTION OF THE INVENTION

The present invention encompasses pharmaceutical compositions comprising candesartan cilexetil, and processes for preparing the same.

It is observed that an amino acid acts as a stabilizer when incorporated as an excipient in a candesartan cilexetil pharmaceutical composition. Typically, amino acids such as L-leucine have been used as a lubricant in tablets of effervescent formulations of alendronate. In these formulations small amounts of lubricants are added to help the tablets, which once pressed are more easily ejected from the die. However, it has now been discovered that amino acids stabilize formulations of pharmaceutical compositions comprising candesartan cilexetil.

Not to be limited by theory, but it is believed that the amino acid absorbs the pressure, abrasion and/or heat during processing, e.g. during a compression step in tabletting a pharmaceutical composition having candesartan cilexetil. Thus, when an amino acid is present in the pharmaceutical composition, it acts as a stabilizer and prevents candesartan cilexetil from decomposing even after it undergoes a compression step. Consequently, the final pharmaceutical composition obtained after processing will contain a less impure candesartan cilexetil.

As used herein, the term "less impure candesartan cilexetil" refers to candesartan cilexetil having about 0.01 % to about 0.5% of desethyl-candesartan ("desethyl-CNS") impurity present in the total weight of the composition, after storage for 4 days at a temperature of 65°C and a relative humidity of 100 percent; and/or having about 0.01% to about 0.5% of 2-N-ethyl candesartan ("2-N-ethyl CNS") impurity present in the total weight of the composition, after storage for 4 days at a temperature of 65°C and a relative humidity of 100 percent; and/or having about 0.01% to about 0.2% of impurities other than desethyl-CNS and 2-N-ethyl CNS present in the total weight of the composition, after storage for 4 days at a temperature of 65°C and a relative humidity of 100 percent.

The structures of the two impurities desethyl-CNS and 2-N-ethyl CNS are as follows:

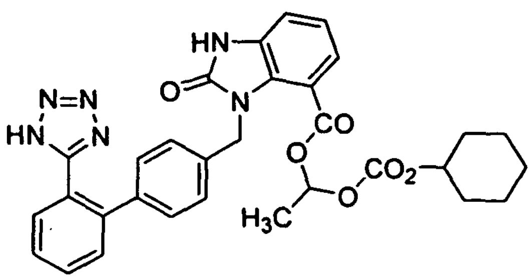

desethyl-CNS

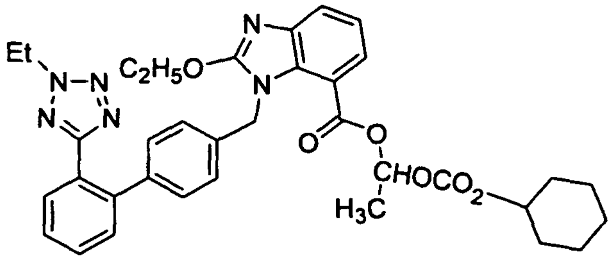

2-N-ethyl CNS

The present invention encompasses a pharmaceutical composition comprising candesartan cilexetil, at least one amino acid and a pharmaceutically acceptable excipient.

Candesartan cilexetil can be prepared using any method known in the art. For example, according to US Publication No. 2005/0250827, substantially pure candesartan cilexetil is prepared by providing cilexetil trityl candesartan, deprotecting cilexetil trityl candesartan in a mixture of water and methanol to obtain a residue of candesartan cilexetil; crystallizing the residue of candesartan cilexetil using methanol and toluene; and recrystallizing the crystalline candesartan cilexetil in methanol to yield a substantially pure candesartan cilexetil.

Typically, the amino acid is a pharmaceutically acceptable amino acid. Preferably, the amino acid is a naturally occurring amino acid selected from the group consisting of alanine, arginine, asparagine, aspartic acid, cysteine, glutamine, glutamic acid, glycine, histidine, isoleucine, leucine, lysine, methionine, phenylalanine, praline, serine, threonine, tryptophan, tyrosine, and valine. More preferably, the amino acid is alanine, valine, leucine or isoleucine. Most preferably, the amino acid is leucine.

Most amino acids can exist in either of two optical isomers, dextrorotatory (D) or levorotary (L). The L-amino acids represent the vast majority of amino acids found in proteins. Consequently, (L)-leucine is the most preferred amino acid used in the present invention.

The amount of amino acid contained in a pharmaceutical composition is not specifically restricted, however, the amount should be sufficient to improve the stability of candesartan cilexetil contained therein. Typically, the amount of amino acid present in a pharmaceutical composition is at about 6% to about 40% by weight of the pharmaceutical composition. Preferably, it is present in an amount of about 10% to about 20% by weight of the pharmaceutical composition and more preferably, it is present in an amount of about 10% by weight of the pharmaceutical composition. In an example embodiment of the present invention, a pharmaceutical composition in the form of a tablet may contain about 2 mg to about 32 mg of amino acid. For instance, a pharmaceutical composition in the form of a tablet may contain 2, 4, 8, 16, 32 mg of candesartan cilexetil. When tablets contain 16 and 32 mg of candesartan cilexetil, an equal amount of amino acid is used to stabilize the candesartan cilexetil.

The pharmaceutical compositions of the present invention may further contain one or more additional excipients including, but not limited to, fillers, diluents, disintegrants, glidants, lubricants, carriers, bulking agents, binders, wetting agents, flavoring agents and the like.

Suitable fillers and diluents include, but are not limited to, cellulose-derived materials like powdered cellulose, microcrystalline cellulose (e.g. Avicele), microfine cellulose, methyl cellulose, ethyl cellulose, hydroxyethyl cellulose, hydroxypropyl cellulose (e.g. Klucel®), hydroxypropyl methylcellulose, carboxymethyl cellulose salts (such as carboxymethyl cellulose calcium) and other substituted and unsubstituted celluloses; starch such as maize starch; pregelatinized starch; lactose, preferably lactose monohydrate (e.g. Pharmatose®); talc; waxes; sugars; sugar alcohols like mannitol and sorbitol; acrylate polymers and copolymers; dextrates; dextrin; dextrose; maltodextrin; pectin; gelatin; inorganic diluents like calcium carbonate, dibasic calcium phosphate dihydrate, tribasic calcium phosphate, calcium sulfate, magnesium carbonate, magnesium oxide, sodium chloride and other diluents known to the pharmaceutical industry.

Most preferred fillers include lactose monohydrate, pregelatinized starch, mannitol or sorbitol. When sugars are used as fillers, the amount of sugar present in a pharmaceutical composition is at about 30%-80% and preferably about 40%-60% by weight of the pharmaceutical composition. When a starch is used as a filler, the amount of starch present in a pharmaceutical composition is about 5%-50% and preferably about 8%-15% by weight of the pharmaceutical composition.

Suitable disintegrants include croscarmellose sodium (e.g. Ac Di Sol®, Primellose®), crospovidone (e.g. Kollidon®, Polyplasdone®), microcrystalline cellulose, polacrilin potassium, powdered cellulose, pregelatinized starch, sodium starch glycolate (e.g. Explotab®, Primoljel®) and starch.

Glidants can be added to improve the flowability of a solid composition before compaction and to improve the accuracy of dosing especially during compaction and capsule filling. Excipients that may function as glidants include colloidal silicon dioxide, magnesium trisilicate, powdered cellulose, and talc.

A lubricant may be added to the pharmaceutical compositions of the present invention to reduce adhesion and/or ease the release of the product from e.g. the die. Suitable lubricants include, but are not limited to, magnesium stearate, calcium stearate, glyceryl monostearate, glyceryl palmitostearate, hydrogenated castor oil, hydrogenated vegetable oil, mineral oil, polyethylene glycol, sodium lauryl sulfate, sodium stearyl fumarate, stearic acid, talc and zinc stearate. Magnesium stearate is most preferred.

Carriers include, but are not limited to, lactose, white sugar, sodium chloride, glucose, urea, starch, calcium carbonate, kaolin, crystalline cellulose, and silicic acid.

Binders include, but are not limited to, carboxymethyl cellulose, shelac, methyl cellulose, potassium phosphate, and polyvinylpyrrolidone. Other suitable binders include, but are not limited to, acacia gum, pregelatinized starch, sodium alginate, glucose and other binders used in wet and dry granulation and direct compression tableting processes.

Flavoring agents and flavor enhancers make the dosage form more palatable to the patient. Common flavoring agents and flavor enhancers for pharmaceutical products that can be included in the composition of the present invention include, but are not limited to, maltol, vanillin, ethyl vanillin, menthol, citric acid, fumaric acid, ethyl maltol, and tartaric acid.

Other excipients that may be incorporated into the formulation include preservatives, surfactants, antioxidants, colourings (e.g. iron oxide red) or any other excipient commonly used in the pharmaceutical industry. Preferably, the pharmaceutical composition may include additional excipients such as, croscarmellose sodium, iron oxide red, spray dried lactose, starch, hydroxypropyl cellulose, sodium lauryl sulfate, microcrystalline cellulose and magnesium stearate.

The pharmaceutical composition may take any form but is preferably a solid composition. More preferably, the pharmaceutical composition of the invention is a compressed solid composition. Suitable solid dosage forms include, but are not limited to, tablets, capsules, powders, or sachets.

Preferably, the dosage form is a tablet. For example, the pharmaceutical composition of the present invention may be a compressed granulate in the form of a tablet or a tablet formed by direct compression or dry granulation of the tablet components. A tablet formed by direct compression is the most preferred.

Many tablets today are coated after being pressed. A coating may be applied to hide the taste of the tablet's components, to make the tablet smoother and easier to swallow, and to make it more resistant to the environment, extending its shelf life. Tablets may be coated with a variety of commonly known coating materials, for example, tablets may be coated with sugar, gelatin film, or enteric coating. There are also double layered tablets and multi-layered tablets.

When tablets and powders are coated with an enteric coating. The enteric coated powder forms may have coatings including, but not limited to, phthalic acid cellulose acetate, hydroxypropylmethyl-cellulose phthalate, polyvinyl alcohol phthalate, carboxymethylethylcellulose, a copolymer of styrene and maleic acid, a copolymer of methacrylic acid and methyl methacrylate, and the like, and if desired, they may be employed with suitable plasticizers and/or extending agents.

When the pharmaceutical composition is in the dosage form of a capsule, the capsule may contain the pharmaceutical composition of the invention in a form of uncompressed or compressed granulates or powder mixes, etc. The capsules may be covered with either a hard shell or a soft shell. The shells may be made from, but not limited to gelatin and optionally contain a plasticizer such as glycerin and sorbitol, and an opacifying agent or colorant.

Methods of administration of a pharmaceutical composition for treating circulatory system diseases of the present invention are not specifically restricted, and can be administered in various preparations depending on the age, sex, and symptoms of the patient. Suitable routes for administrating a pharmaceutical composition may include, but not limited to, oral, buccal, and rectal administration. Although the most suitable administration in any given case will depend on the nature and severity of the condition being treated, the most preferred route of administration of the present

invention is oral.

The dosages may be conveniently presented in unit dosage form and prepared by any of the methods well known in the pharmaceutical arts. The pharmaceutical compositions of the present invention may be prepared by any conventional means such as, but not limited to, wet or dry granulation and direct compression. Preferably, the pharmaceutical compositions are prepared by direct compression.

The amount of candesartan cilexetil contained in a pharmaceutical composition for treating circulatory system diseases according to the present invention is not specifically restricted, however, the dose should be sufficient to treat, ameliorate, or reduce the symptoms associated with the circulatory system disease. The dosage of a pharmaceutical composition for treating circulatory system diseases according to the present invention will depend on the method of use, the age, sex, and condition of the patient. Preferably, about 2 mg to 32 mg of candesartan cilexetil may be contained in an administration form unit.

In one embodiment, the present invention encompasses a process of preparing a pharmaceutical composition comprising combining candesartan cilexetil, at least one amino acid and a pharmaceutically acceptable excipient.

Additional excipients such as those described above may be combined with the candesartan cilexetil and the amino acid. The excipients may be added at the same time as candesartan cilexetil and the amino acid are combined. Optionally, additional excipients may be introduced after candesartan cilexetil and the amino acid are combined.

The process of the invention further comprises at least one compression step. Preferably, the compression step is a direct compression process. In a direct compression process, the process of the invention comprises combining candesartan cilexetil and an amino acid, and optionally one or more excipients, and compressing the resultant combination into a solid pharmaceutical composition, preferably a tablet.

Optionally, the compression step includes a mixture made by dry granulation or direct compression.

Typically, the amino acid is a pharmaceutically acceptable amino acid. Preferably, a naturally occurring amino acid selected from the group consisting of alanine, arginine, asparagine, aspartic acid, cysteine, glutamine, glutamic acid, glycine, histidine, isoleucine, leucine, lysine, methionine, phenylalanine, praline, serine, threonine, tryptophan, tyrosine, and valine. More preferably, the amino acid is (L)-leucine.

Having described the invention with reference to certain preferred embodiments, other embodiments will become apparent to one skilled in the art from consideration of the specification. The invention is further defined by reference to the following examples describing in detail the pharmaceutical compositions and processes for preparing the pharmaceutical compositions of the invention. It will be apparent to those skilled in the art that many modifications, both to materials and methods, may be practiced without departing from the scope of the invention.

## EXAMPLES

### Example 1

A pharmaceutical composition according to the present invention was prepared in the form of a tablet.

Croscarmellose sodium (Primellose®) (6.4 mg, 2% w/w) and iron red oxide (0.3 mg, 0.09 % w/w) were sieved through a #80 mesh sieve. The resultant mixture was loaded into a Y-cone together with candesartan cilexetil (32.0 mg, 10% w/w), lactose monohydrate (Pharmatose®) (180.0 mg, 56% w/w), starch (starch 1500) (34.0 mg, 11% w/w), hydroxypropyl cellulose (Klucel® L) (9.6 mg, 3% w/w), (L)-leucine (32.0 mg, 10% w/w), sodium lauryl sulfate (1.6 mg, 0.5% w/w), and microcrystalline cellulose (Avicel® PH 102) (22.4 mg, 7% w/w) and mixed for 5 minutes. The resultant blend was sieved through a #0.032 inch sieve and loaded into a Y-cone. Magnesium stearate (1.7 mg, 0.5% w/w) was sieved through a #50 mesh sieve and also loaded into the Y-cone. Mixing was carried out for 5 minutes. The final blend was compressed into tablets.

### Example 2

A pharmaceutical composition according to the present invention was prepared in the form of a tablet.

Croscarmellose sodium (Primellose®) (6.4 mg, 2% w/w) and iron red oxide (0.3 mg, 0.09 % w/w) were sieved through a #80 mesh sieve. The resultant mixture was loaded into a Y-cone together with candesartan cilexetil (32.0 mg, 10% w/w), lactose monohydrate (Pharmatose®) (184.8 mg, 57.75% w/w), starch (starch 1500) (34.0 mg, 11 % w/w), hydroxypropyl cellulose (Klucel® L) (6.4 mg, 2% w/w), (L)-leucine (32.0 mg, 10% w/w) and microcrystalline cellulose (Avicel® PH 102) (22.4 mg, 7% w/w) and mixed for 5 minutes. The resultant blend was sieved through a #0.032 inch sieve and loaded into a Y-cone. Magnesium stearate (1.7 mg, 0.5% w/w) was sieved through a #50 mesh sieve and also loaded into the Y-cone. Mixing was carried out for 5 minutes. The final blend was compressed into tablets.

HPLC

Typically, side products, by-products, and adjunct reagents (collectively defined as "impurities") are identified spectroscopically and/or with another physical method, and then associated with a peak position, such as that in a chromatogram, or a spot on a TLC plate. (Strobel, H.A. and Heineman, W.R., CHEMICAL INSTRUMENTATION: A SYSTEMATIC APPROACH, p. 953, 3rd dd. (Wiley & Sons: New York 1989)). Thereafter, the impurity can be identified, e.g., by its relative position in the chromatogram, where the position in a chromatogram is conventionally measured in minutes between injection of the sample on the column and elution of the particular component through the detector. The relative position in the chromatogram is known as the "retention time."

The retention time can vary about a mean value based upon the condition of the instrumentation, as well as many other factors. To mitigate the effects such variations have upon accurate identification of an impurity, practitioners use the "relative retention time" ("RRT") to identify impurities. (Strobel p. 922). The RRT of an impurity is its retention time divided by the retention time of a reference marker. It may be advantageous to select a compound other than the API that is added to, or present in, the mixture in an amount sufficiently large to be detectable and sufficiently low as not to saturate the column, and to use that compound as the reference marker for determination of the RRT.

The stability of the tablet prepared in each Examples 1 and 2 was measured by high performance liquid chromatography (HPLC). HPLC was performed on a YMC-pack C8 analytical column (4.6 x 100 mm), packed with S-3μm or equivalent particles (YMC Co., Ltd.). The mobile phase was a mixture of solutions A and B. Solution A was a solution of phosphoric acid (at pH 2.1 adjusted with water) and acetonitrile (65:35). Solution B was a solution of phosphoric acid (at pH 2.1 adjusted with water) and acetonitrile (20:80). The flow-rate was 1.5 ml/min and UV detector was set at 254 nm. The column temperature was 40°C. The diluent was a mixture of water and acetonitrile (50:50).

The standard solutions were prepared as follows: Standard solution A was prepared by accurately weighing 16 mg of candesartan cilexetil A.S. into a 50 ml volumetric flask and dissolved it in 25 ml acetonitrile. Then to it was added 15 ml of water, mixed, adjusted to room temperature and diluted to 0.32 mg/ml with water. Standard solution B (0.1 %) was prepared by accurately pipetting 2.5 ml of standard solution A into a 100 ml volumetric flask and made up to volume with diluent. Diluted 2.0 ml of the obtained solution to 50 ml with diluent (concentration at 0.3 μg/ml). RL solution (0.05%) was prepared by accurately pipetting 2.5 ml of standard solution B into a 5 ml volumetric flask and made up to volume with diluent (concentration at 0.15 μg/ml).

The sample solutions were prepared as follows: Accurately weighed NLT (not less than) 20 tablets and the average tablet weight was calculated. Then accurately weighed (n) tablets into (V) ml volumetric flask according to the following table:

| Strength, mg | Amount of tablets (n) | Volume of the volumetric flask (V) |
|---|---|---|
| 2 | 8 | 50 |
| 4 | 8 | 100 |
| 8 | 8 | 200 |
| 16 | 5 | 250 |
| 32 | 5 | 500 |

Water was then added at approximately 0.2 V of the flask volume and shook for 30 minutes up to complete tablets disintegration. Acetonitrile was then added at approximately 0.5 V of the flask volume and shook for 1 hr. Made up to volume with water and mixed well. Filtered through 0.45 μm membrane filter and discarded first 4-5 ml.

The system suitability test must conform to the following criteria: The typical retention time of the candesartan cilexetil peak was approximately 18-24 minutes. The tailing factor of the candesartan cilexetil peak should be in the range 0.7-1.5. The resolution between the candesartan cilexetil and the peaks of the thermodegradation products (RRt 0.9 and RRt 1.1) eluting both before and after it should not be less than 2.0. Identified in the obtained chromatogram are peaks of the CNS acid, CNS ethyl ester, CNS desethyl, CNS 2N-ethyl and degradation products RRt 0.9 and RRt 1.1. The system suitability test was performed by injecting the system with a suitability solution. The system suitability solution was prepared as follows: An aliquot of the standard solution A was heated in a tightly closed microreaction vessel with screw head at 110°C for 1 hour and allowed to cool down. To 10 ml of this solution, added 0.1 ml of each of the marker solutions and mixed well. Marker solutions were prepared as follows: Marker solution of CNS acid was prepared by weighing approximately 3 mg of the CNS acid A.S. into a 20 ml volumetric flask. To that, 10 ml of acetonitrile and 5 ml of water were added, then dissolved and diluted to volume with water (concentration at 0.15mg/ml). Marker solution of candesartan ethyl ester was prepared by weighing approximately 3 mg of the CNS ethyl ester A.S. into a 20

ml volumetric flask. To that, 10 ml of acetonitrile and 4 ml of water were added, then dissolved and diluted to volume with acetonitrile (concentration at 0.15 mg/ml).

The procedure for measuring the stability of the tablet prepared in each Examples 1 and 2 was performed as follows: The system suitability compliance was checked. The diluent solution was injected to observe any system peaks. The RL standard solution was injected once. The integration parameters were set to detect any peak area that is NLT half an area of the candesartan peak in this chromatogram. One of the standard solutions B was injected six times. The relative standard deviation of the candesartan peak areas of replicate injections should not be more than 10.0%. The second standard solution B was injected NMT (not more than) twice. The relative difference between the averages of the normalized peak areas obtained for the two standard solutions B should be NMT 15%. One of the standard solutions A was injected five times. The relative standard deviation of the candesartan peak areas in the replicate injections should not be more than 2.0%. The second assay standard solution was injected NMT twice. The relative difference between the averages of the normalized peak areas obtained for the two assay standard solutions should be NMT 15%. After every 6 sample solution injections, one of the standard solutions was injected as a control.

Assay and impurity/degradation product were calculated as follows.

For assay: % candesartan cilexetil of labeled amount = {[sample peak area x sample volume (ml) x average tablet weight (mg)]/[sample weight (mg) x strength (mg) (in terms of candesartan cilexetil)]} x {[standard weight (mg) (take into account the % assay and the % water of the standard)]/[standard A volume (ml) x standard A average peak area]} x 100

For impurities and degradation products: % impurity/degradation product related to candesartan cilexetil = {[impurity peak area x sample volume (ml) x average tablet weight (mg)]/[sample weight (mg) x strength (mg) (in terms of candesartan cilexetil)]} x {[standard weight (mg) (take into account the % assay and the % water of the standard)]/[standard volume (ml) (take into account dilution) x standard B average peak area x RRF (relative response factor)]} x 100

Take RRF equal 1.0 for all identified and not identified impurities/degradation products.

Reporting Limit (RL): Area corresponding to RL (0.05%) = [Mean standard B (0.1%) area/2] x RRF

The procedure for reporting the stability of tablets prepared from Examples 1 and 2 is as follows: Calculated and reported precise percentage for any impurity/degradation peaks with areas equivalent to, or greater than, the area corresponding to the RL concentration (0.05%). Known peaks were identified by name. For unknown peaks, their relative retention times were reported. If no additional peaks were observed, or for any peak observed with a peak area less than the RL, then it was reported "less than 0.05%". The total percentage of impurities and degradation products were reported.

Results

The results for the stability of tablets prepared from examples 1 and 2 are presented in Tables 1 and 2. For comparison, the stability of the commercially available Atacand® tablet was also measured by HPLC and the results are presented in Table 3. The Atacand® tablet is identified by United States N.D.A. Number 20-838 (strength 32 mg).

Table 1: Tablet prepared in Example 1.

| Time(days) | Degradation products Assay % | | | | |
|---|---|---|---|---|---|
| | desethyl-CNS | *RRt 0.9 | *RRt 1.1 | *RRt 1.2 | 2-N-ethyl CNS |
| 0 | - | - | - | - | - |
| 4 | 0.3 | - | 0.1 | 0.08 | 0.2 |
| *RRt = relative retention time (relative to the retention time of candesartan cilexetil) | | | | | |

Table 2: Tablet prepared in Example 2.

| Time(days) | Degradation products Assay % | | | | |
|---|---|---|---|---|---|
| | desethyl-CNS | *RRt 0.9 | *RRt 1.1 | *RRt 1.2 | 2-N-ethyl CNS |
| 0 | 0.07 | - | - | - | 0.07 |
| 4 | 0.3 | 0.07 | 0.2 | 0.09 | 0.2 |
| *RRt = relative retention time (relative to the retention time of candesartan cilexetil) | | | | | |

Table 3: Commercially available Atacand® tablet (32 mg).

| Time(days) | Degradation products Assay % | | | | |
|---|---|---|---|---|---|
| | desethyl-CNS | *RRt 0.9 | *RRt 1.1 | *RRt 1.2 | 2-N-ethyl CNS |
| 0 | 0.2 | - | - | 0.08 | 0.2 |
| 4 | 0.7 | 0.09 | 0.2 | 0.2 | 0.5 |
| *RRt = relative retention time (relative to the retention time of candesartan cilexetil) | | | | | |

The results in Tables 1-3 demonstrate that the tablet produced according to the present invention exhibits improved stability in comparison to the commercially available Atacand® tablet. The impurity levels of desethyl-CNS and 2-N-ethyl CNS found in the tablet made with the composition of the invention are considerably lower than those found in the commercially available Atacand® tablet after 4 days in storage.

**Claims**

1. A pharmaceutical composition comprising candesartan cilexetil, at least one amino acid and a pharmaceutically acceptable excipient.

2. The pharmaceutical composition of claim 1, wherein the candesartan cilexetil has 0.01% to 0.5% of desethyl-candesartan present in the total weight of the composition, after the composition is stored for four days at a temperature of about 65°C with a relative humidity of 100 percent.

3. The pharmaceutical composition of claims 1 or 2, wherein the candesartan cilexetil has 0.01% to 0.5% of 2-N-ethyl candesartan present in the total weight of the composition, after the composition is stored for four days at a temperature of 65°C with a relative humidity of 100 percent.

4. The pharmaceutical composition according to any one of claims 1-3, wherein the candesartan cilexetil has 0.01 % to 0.2% of other impurities other than desethyl-candesartan and 2-N-ethyl candesartan present in the total weight of the composition, after the composition is stored for four days at a temperature of 65°C with a relative humidity of 100 percent.

5. The pharmaceutical composition according to any one of claims 1-4, wherein the amino acid is a naturally occurring amino acid selected from the group consisting of alanine, arginine, asparagine, aspartic acid, cysteine, glutamine,

glutamic acid, glycine, histidine, isoleucine, leucine, lysine, methionine, phenylalanine, praline, serine, threonine, tryptophan, tyrosine, and valine, preferably wherein the amino acid is alanine, valine, leucine or isoleucine, and most preferably (L)-leucine.

**6.** The pharmaceutical composition according to any one of claims 1-5, wherein the amino acid present is at 6% to 40%, preferably 10% to 20%, by weight of the pharmaceutical composition.

**7.** The pharmaceutical composition according to any one of claims 1-6, wherein the excipient is selected from the group consisting of croscarmellose sodium, iron oxide red, spray dried lactose, starch, hydroxypropyl cellulose, sodium lauryl sulfate, microcrystalline cellulose and magnesium stearate.

**8.** A process for preparing a pharmaceutical composition comprising:

combining candesartan cilexetil, at least one amino acid and a pharmaceutically acceptable excipient.

**9.** The process of claim 8, wherein the amino acid is a naturally occurring amino acid selected from the group consisting of alanine, arginine, asparagine, aspartic acid, cysteine, glutamine, glutamic acid, glycine, histidine, isoleucine, leucine, lysine, methionine, phenylalanine, praline, serine, threonine, tryptophan, tyrosine, and valine, preferably (L)-leucine.

**10.** The process according to claim8 or claim 9, wherein the amino acid present is at 6% to 40%, preferably 10% to 20%, by weight of the pharmaceutical composition.

**11.** The process according to any one of claims 8-10, wherein the excipient is selected from the group consisting of croscarmellose sodium, iron oxide red, spray dried lactose, starch, hydroxypropyl cellulose, sodium lauryl sulfate, microcrystalline cellulose and magnesium stearate.

**12.** The process according to any one of claims 8-11, wherein the process further comprises a compression step, preferably wherein the compression step is a direct compression process.

**13.** The process according to any one of claims 8-12, wherein the pharmaceutical composition is a solid composition.

**14.** The use of at least one amino acid for the stabilization of candesartan cilexetil in a pharmaceutical composition.

**15.** The pharmaceutical composition comprising candesartan cilexetil, at least one amino acid and a pharmaceutically acceptable excipient as defined in any one of claims 1-7 for use in the treatment of a patient suffering from a circulatory system disease, preferably wherein the disease is hypertension.

## Patentansprüche

**1.** Pharmazeutische Zusammensetzung, umfassend Candesartan-Cilexetil, mindestens eine Aminosäure und einen pharmazeutisch zulässigen Hilfsstoff.

**2.** Pharmazeutische Zusammensetzung nach Anspruch 1, wobei das Candesartan-Cilexetil nach Lagerung der Zusammensetzung für vier Tage bei einer Temperatur von etwa 65°C bei einer relativen Luftfeuchtigkeit von 100 Prozent einen Desethyl-Candesartan-Anteil von 0,01 % bis 0,5 % des Gesamtgewichts der Zusammensetzung aufweist.

**3.** Pharmazeutische Zusammensetzung nach den Ansprüchen 1 oder 2, wobei das Candesartan-Cilexetil nach Lagerung der Zusammensetzung für vier Tage bei einer Temperatur von 65°C bei einer relativen Luftfeuchtigkeit von 100 Prozent einen 2-N-Ethyl-Candesartan-Anteil von 0,01 % bis 0,5 % des Gesamtgewichts der Zusammensetzung aufweist.

**4.** Pharmazeutische Zusammensetzung nach einem der Ansprüche 1 bis 3, wobei das Candesartan-Cilexetil nach Lagerung der Zusammensetzung für vier Tage bei einer Temperatur von 65°C bei einer relativen Luftfeuchtigkeit von 100 Prozent einen Anteil anderer Verunreinigungen als Desethyl-Candesartan und 2-N-Ethyl-Candesartan von 0,01 % bis 0,2 % des Gesamtgewichts der Zusammensetzung aufweist.

5. Pharmazeutische Zusammensetzung nach einem der Ansprüche 1 bis 4, wobei die Aminosäure eine natürlich vorkommende Aminosäure ist, ausgewählt aus der Gruppe bestehend aus Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin und Valin, wobei die Aminosäure vorzugsweise Alanin, Valin, Leucin oder Isoleucin ist und am meisten bevorzugt (L)-Leucin.

6. Pharmazeutische Zusammensetzung nach einem der Ansprüche 1 bis 5, wobei die vorhandene Aminosäure 6 bis 40 Gew.-%, vorzugsweise 10 bis 20 Gew.-% der pharmazeutischen Zusammensetzung ausmacht.

7. Pharmazeutische Zusammensetzung nach einem der Ansprüche 1-6, wobei der Hilfsstoff ausgewählt ist aus der Gruppe bestehend aus Croscarmellose-Natrium, Eisenoxidrot, sprühgetrockneter Laktose, Stärke, Hydroxypropylcellulose, Natriumlaurylsulfat, mikrokristalliner Cellulose und Magnesiumstearat.

8. Verfahren zur Herstellung einer pharmazeutischen Zubereitung, umfassend:

   das Kombinieren von Candesartan-Cilexetil, mindestens einer Aminosäure und einem pharmazeutisch zulässigen Hilfsstoff.

9. Verfahren nach Anspruch 8, wobei die Aminosäure eine natürlich vorkommende Aminosäure ist, ausgewählt aus der Gruppe bestehend aus Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin und Valin, bevorzugt (L)-Leucin.

10. Verfahren nach Anspruch 8 oder 9, wobei die vorhandene Aminosäure 6 bis 40 Gew.-% , vorzugsweise 10 bis 20 Gew.-% der pharmazeutischen Zusammensetzung ausmacht.

11. Verfahren nach einem der Ansprüche 8 bis 10, wobei der Hilfsstoff ausgewählt ist aus der Gruppe bestehend aus Croscarmellose-Natrium, Eisenoxidrot, sprühgetrockneter Laktose, Stärke, Hydroxypropylcellulose, Natriumlaurylsulfat, mikrokristalliner Cellulose und Magnesiumstearat.

12. Verfahren nach einem der Ansprüche 8 bis 11, wobei das Verfahren ferner einen Verpressungsschritt umfasst, wobei der Verpressungsschritt vorzugsweise ein direktes Verpressungsverfahren ist.

13. Verfahren nach einem der Ansprüche 8 bis 12, wobei die pharmazeutische Zusammensetzung eine feste Zusammensetzung ist.

14. Verwendung mindestens einer Aminosäure zur Stabilisierung von Candesartan-Cilexetil in einer pharmazeutischen Zusammensetzung.

15. Pharmazeutische Zusammensetzung, umfassend Candesartan-Cilexetil, mindestens eine Aminosäure und einen pharmazeutisch zulässigen Hilfsstoff, wie in einem der Ansprüche 1 bis 7 definiert, zur Verwendung bei der Behandlung eines Patienten, der an einer Erkrankung des Kreislaufsystems leidet, wobei die Erkrankung vorzugsweise Hypertonie ist.

## Revendications

1. Composition pharmaceutique comprenant du candesartan cilexetil, au moins un acide aminé et un excipient pharmaceutiquement acceptable.

2. Composition pharmaceutique selon la revendication 1, dans laquelle le candesartan cilexetil a une proportion de 0,01 % à 0,5% de deséthyl-candesartan dans le poids total de la composition, après que la composition a été stockée pendant quatre jours à une température d’environ 65°C à une humidité relative de 100 pour cent.

3. Composition pharmaceutique selon la revendication 1 ou 2, dans laquelle le candesartan cilexetil a une proportion de 0,01% à 0,5% de 2-N-éthyl-candesartan dans le poids total de la composition, après que la composition a été stockée pendant quatre jours à une température d’environ 65°C à une humidité relative de 100 pour cent.

**4.** Composition pharmaceutique selon l'une quelconque des revendications 1-3, dans laquelle le candesartan cilexetil a une proportion de 0,01% à 0,2% d'autres impuretés différentes du deséthyl-candesartan et du 2-N-éthyl candesartan dans le poids total de la composition, après que la composition a été stockée pendant quatre jours à une température d'environ 65°C à une humidité relative de 100 pour cent.

**5.** Composition pharmaceutique selon l'une quelconque des revendications 1-4, dans laquelle l'acide aminé est un acide aminé existent par nature sélectionné dans le groupe constitué de l'alanine, l'arginine, l'asparagine, l'acide aspartique, la cystéine, la glutamine, l'acide glutamique, la glycine, l'histidine, l'isoleucine, la leucine, la lysine, la méthionine, la phénylalanine, la praline, la sérine, la thréonine, le tryptophane, la tyrosine, et la valine, de préférence dans laquelle l'acide aminé est l'alanine, la valine, la leucine ou l'isoleucine, et le plus préférablement la (L)-leucine.

**6.** Composition pharmaceutique selon l'une quelconque des revendications 1-5, dans laquelle l'acide aminé est présent à une proportion de 6% à 40%, de préférence 10% à 20% en poids de la composition pharmaceutique.

**7.** Composition pharmaceutique selon l'une quelconque des revendications 1-6, dans laquelle l'excipient est sélectionné dans le groupe constitué de croscarmellose sodium, oxyde de fer rouge, lactose séché par pulvérisation, amidon, cellulose hydroxypropyle, laurylsulfate de sodium, cellulose microcristalline et stéarate de magnésium.

**8.** Procédé pour la production d'une composition pharmaceutique, comprenant :

la combinaison de candesartan cilexetil, au moins un acide aminé et un excipient pharmaceutiquement acceptable.

**9.** Procédé selon la revendication 8, dans lequel l'acide aminé est un acide aminé existent par nature sélectionné dans le groupe constitué de l'alanine, l'arginine, l'asparagine, l'acide aspartique, la cystéine, la glutamine, l'acide glutamique, la glycine, l'histidine, l'isoleucine, la leucine, la lysine, la méthionine, la phénylalanine, la praline, la sérine, la thréonine, le tryptophane, le tyrosine, et la valine, de préférence la (L)-leucine.

**10.** Procédé selon la revendication 8 ou la revendication 9, dans laquelle l'acide aminé est présent à une proportion de 6% à 40%, de préférence 10% à 20% en poids de la composition pharmaceutique.

**11.** Procédé selon l'une quelconque des revendications 8-10, dans laquelle l'excipient est sélectionné dans le groupe constitué de croscarmellose sodium, oxyde de fer rouge, lactose séché par pulvérisation, amidon, cellulose hydroxypropyle, laurylsulfate de sodium, cellulose microcristalline et stéarate de magnésium.

**12.** Procédé selon l'une quelconque des revendications 8-11, dans lequel le procédé comprend en outre une étape de compression, de préférence dans lequel l'étape de compression est un procédé de compression directe.

**13.** Procédé selon l'une quelconque des revendications 8-12, dans lequel la composition pharmaceutique est une composition solide.

**14.** Utilisation d'au moins un acide aminé pour la stabilisation de candesartan cilexetil dans une composition pharmaceutique.

**15.** Composition pharmaceutique comprenant du candesartan cilexetil, au moins un acide amine et un excipient pharmaceutiquement acceptable comme défini dans l'une quelconque des revendications 1-7 pour utilisation dans le traitement d'un patient souffrant d'une maladie du système circulatoire, de préférence dans laquelle la maladie est l'hypertension.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 97044017 A
- US 4755461 A
- EP 0459136 A
- EP 0546358 A
- US 20050250827 A


### Non-patent literature cited in the description


- **Strobel, H.A. ; Heineman, W.R.** CHEMICAL INSTRUMENTATION: A SYSTEMATIC APPROACH. Wiley & Sons, 1989, 953